# EUROPÄISCHE PATENTSCHRIFT

(11) **EP 2 442 854 B1**
(45) Veröffentlichungstag und Bekanntmachung des Hinweises auf die Patenterteilung: **12.08.2015**
(21) Anmeldenummer: 10725035.9
(22) Anmeldetag: 09.06.2010
(51) Int. Cl.: A61M 5/315, A61M 5/20, A61M 5/178

(54) **KOLBEN FÜR EINEN MEDIZINISCHEN HOHLKÖRPER UND MEDIZINISCHER HOHLKÖRPER**
PISTON FOR A MEDICAL HOLLOW CONTAINER AND MEDICAL HOLLOW CONTAINER
PISTON POUR CORPS CREUX À USAGE MÉDICAL ET CORPS CREUX À USAGE MÉDICAL

(30) Priorität: 18.06.2009 DE 102009025375
(43) Veröffentlichungstag der Anmeldung: 25.04.2012
(60) Teilanmeldung: 15166338.2
(73) Patentinhaber: Arzneimittel GmbH Apotheker Vetter & Co. Ravensburg, 88212 Ravensburg (DE)
(72) Erfinder: BOETTGER, Frank, 88214 Ravensburg (DE); BOEBST, Benjamin, 88441 Mittelbiberach (DE)
(74) Vertreter: Gleiss, Alf-Olav
(86) Internationale Anmeldenummer: PCT/EP2010/003449
(87) Internationale Veröffentlichungsnummer: WO 2010/145771

(56) Entgegenhaltungen:
- EP-A1- 1 317 937
- US-A- 1 949 612
- US-A- 2 545 017
- US-A- 5 353 691

## Beschreibung

Die Erfindung betrifft einen Kolben für einen medizinischen Hohlkörper gemäß Oberbegriff des Anspruchs 1. Sie betrifft außerdem einen Kolben für einen medizinischen Hohlkörper gemäß Oberbegriff des Anspruchs 11.

Kolben für medizinische Hohlkörper, insbesondere Spritzen oder Karpulen, sind bekannt. Sie weisen typischerweise eine im Wesentlichen zylindrische Form auf und sind in einen ebenfalls im Wesentlichen zylindrischen Innenraum eines medizinischen Hohlkörpers einbringbar. An einer äußeren Mantelfläche weist ein solcher Kolben mindestens eine Gleitfläche auf, mit der er an einer inneren Mantelfläche eines Innenraums des Hohlkörpers anliegt. Vorzugsweise umfasst der Kolben zumindest im Bereich dieser Gleitfläche ein Material, das elastischer ist als das Material, aus dem der medizinische Hohlkörper besteht. Außerdem ist vorzugsweise der Außendurchmesser des Kolbens geringfügig größer als der Innendurchmesser des medizinischen Hohlkörpers. Wird der Kolben so in den medizinischen Hohlkörper eingebracht, dass die Längsachsen des Kolbens und des Hohlkörpers miteinander fluchten, ergibt sich so eine zumindest geringfügige Kompression des Kolbenmaterials im Bereich der mindestens einen Gleitfläche, sodass hier eine Dichtwirkung entsteht. Der Kolben verschließt also den Innenraum des Hohlkörpers dichtend. Gleichzeitig ist eine Verlagerung des Kolbens in dem Hohlkörper möglich, wobei der Kolben mit seiner mindestens einen Gleitfläche an der inneren Mantelfläche des Hohlkörpers gleitet. Diese Gleitbewegung des Kolbens kann dazu genutzt werden, ein Medium, beispielsweise eine pharmazeutische Substanz oder eine Probe durch eine dem Kolben - in axialer Richtung des Hohlkörpers gesehen - gegenüberliegende Öffnung des Hohlkörpers wahlweise auszutreiben oder einzuziehen.

Bekannte Kolben für medizinische Hohlkörper umfassen Materialien, die in Verbindung mit dem Material, aus dem der Hohlkörper besteht, im Bereich der mindestens einen Gleichfläche des Kolbens bei Anlage desselben an dem Hohlkörper hohe Haft- und Gleitreibungskoeffizienten aufweisen, sodass eine Verlagerung des Kolbens relativ zu dem Hohlkörper ohne weitere Maßnahmen mit hinnehmbarem Kraftaufwand nicht möglich ist. Je nach Elastizität des Kolbens kann dieser sich bei Einleitung von Axialkräften verformen, was zu einer Erhöhung der Haftreibung im Bereich der mindestens einen Gleitfläche führt. Eine Erhöhung der Axialkräfte führt dann dazu, dass die einer Verlagerung des Kolbens entgegenwirkenden Reibungskräfte verstärkt werden, sodass letztendlich eine Verblockung des Kolbens auftritt, dieser also gar nicht mehr verlagert werden kann. Bei weiterer Erhöhung der Axialkräfte kann es zu einer Zerstörung des Kolbens kommen.

Um eine Verlagerung des Kolbens bei hinnehmbarem Kraftaufwand unter Vermeidung einer Verblockung zu ermöglichen, wird die innere Mantelfläche des Hohlkörpers bekannterweise mit einem Gleitmittel beschichtet, sodass der Kolben bei seiner Verlagerung in dem Hohlkörper mit seiner mindestens einen Gleitfläche auf einem durch das Gleitmittel gebildeten Schmierfilm gleiten kann. Als Gleitmittel finden typischerweise Silikon, Silikonöl und/oder Silikonölemulsionen Anwendung.

Um eine Verlagerung des Kolbens entlang der gesamten axialen Erstreckung des Innenraums des Hohlkörpers zu ermöglichen, muss dessen gesamte innere Mantelfläche mit dem Gleitmittel beschichtet werden. Dies erfolgt üblicherweise vor einem Einbringen des Kolbens in den Hohlkörper und insbesondere vor einem Befüllen des Hohlkörpers mit einer pharmazeutischen Substanz. Hierdurch steht während einer Lagerung des vorgefüllten Hohlkörpers die pharmazeutische Substanz in Kontakt mit dem Gleitmittel. Nachteilig hieran ist, dass es insbesondere bei biotechnologisch hergestellten, sensiblen pharmazeutischen Substanzen zu Wechselwirkungen mit dem Gleitmittel kommen kann. Insbesondere ist bekannt, dass Silikonöl zur Instabilität von Wirkstoffen führen kann, die Proteine und/oder Peptide umfassen. Beispielsweise kann es zu einer Aggregatbildung oder Niederschlagsfällung kommen. Die sich gegebenenfalls bildenden Aggregate stehen auch unter Verdacht, eine Reihe unerwünschter Immunreaktionen auszulösen. Die Beschichtung des Innenraums eines medizinischen Hohlkörpers, der eine zur Injektion in einen Patienten bestimmte Substanz umfasst, kann also nicht nur zu einer Herabsetzung der Wirksamkeit des Wirkstoffs führen, sondern durchaus auch negative Folgen für die Gesundheit des Patienten haben, dem die Injektion verabreicht wird.

Aus dem Stand der Technik, offenbart im Dokument US 5 353 691 A, ist schließlich auch ein Kolben für einen medizinischen Hohlkörper mit mindestens einer Gleitfläche und mit mindestens einem Aufnahmebereich, in dem mindestens ein Gleitmittel aufnehmbar ist, bekannt Weiterhin ist mindestens ein Abgabemittel vorgesehen, durch welches das mindestens eine Gleitmittel von dem mindestens einen Aufnahmebereich an die mindestens eine Gleitfläche des Kolbens abgebbar ist. Der Kolben umfasst also selbst das zu seiner Verlagerung benötigte Gleitmittel und verteilt es mit Hilfe des Abgabemittels vorzugsweise während seiner Verlagerung in dem Bereich der mindestens einen Gleitfläche, sodass hier ein Schmierfilm entsteht, auf dem der Kolben gleiten kann. Der Schmierfilm wird so zu jedem Zeitpunkt der Verlagerung des Kolbens gerade dort erzeugt, wo sich der Kolben befindet. Der Oberbegriff des Anspruchs 1 ist auf in diesem Dokument offenbartem Gegenstand basiert.

Es hat sich herausgestellt, dass das Gleitmittel unter Umständen die in dem medizinischen Hohlkörper vorhandene pharmazeutische Substanz während der Lagerung des Hohlkörpers beeinträchtigt.

Aufgabe der Erfindung ist es daher, einen Kolben für einen medizinischen Hohlkörper zu schaffen, bei dem eine Beeinträchtigung der pharmazeutischen Substanz in den medizinischen Hohlkörper mit hoher Sicherheit vermieden wird.

Zur Lösung dieser Aufgabe wird ein Kolben vorgeschlagen, der die Merkmale des Anspruchs 1 aufweist. Er zeichnet sich dadurch aus, dass er mit einer Sperreinrichtung zusammenwirkt, und dass in einem ersten Zustand des Kolbens kein Gleitmittel aus mindestens einem Aufnahmebereich abgebbar ist, und dass in einem zweiten Zustand des Kolbens Gleitmittel durch mindestens ein Abgabemittel an die wenigstens eine Gleitfläche des Kolbens abgebbar ist.

Durch diese Ausgestaltung des Kolbens ist gewährleistet, dass in einem ersten Zustand kein Gleitmittel aus dem mindestens einen Aufnahmebereich abgebbar ist, und dass in einem zweiten Zustand des Kolbens Gleitmittel durch das mindestens eine Abgabemittel an die mindestens eine Gleitfläche abgebbar ist. Befindet sich der Kolben also in seinem ersten Zustand, was typischerweise während der Lagerung des mit einer pharmazeutischen Substanz vorgefüllten und mit dem Kolben versiegelten Hohlkörpers der Fall ist, kann kein Gleitmittel aus dem Aufnahmebereich abgegeben werden, sodass insbesondere kein Kontakt des Gleitmittels mit der pharmazeutischen Substanz möglich ist. Durch das Gleitmittel verursachte. Instabilitäten der pharmazeutischen Substanz können so vermieden werden.

Der zweite Zustand spricht eine Verlagerung des Kolbens, also beispielsweise eine Injektion an. In diesem Zustand soll Gleitmittel an die mindestens eine Gleitfläche abgegeben werden, um eine möglichst reibungsarme Verlagerung des Kolbens zu ermöglichen.

Bevorzugt wird ein Ausführungsbeispiel, bei dem der Kolben in dem ersten Zustand drucklos und in dem zweiten Zustand mit einem Druck beaufschlagt ist. Solange der Kolben nicht mit einem Druck beaufschlagt wird, bleibt das Gleitmittel in dem Aufnahmebereich, während der auf den Kolben ausgeübte Druck in dem zweiten Zustand zum Einen eine Abgabe des Gleitmittels und zum Anderen eine Verlagerung des Kolbens bewirkt.

Weitere vorteilhafte Ausgestaltungen ergeben sich aus den Unteransprüchen.

Im Folgenden wird noch ein nicht zur Erfindung gehöriger Kolben erwähnt, der zumindest im Bereich der mindestens einen Gleitfläche ein glattes, unpolares Material umfasst. Ein solches Material verleiht dem Kolben im Vergleich zu bekannten Kolben eine geringere Haftreibung beziehungsweise auch eine verringerte Gleitreibung in Kontakt mit der inneren Mantelfläche des Hohlkörpers. Auch in diesem Fall kann also auf eine Beschichtung der inneren Mantelfläche des Hohlkörpers mit einem Gleitmittel verzichtet werden, ohne dass deshalb übermäßig hohe Kräfte zur Verlagerung des Kolbens in dem Hohlkörper aufgewendet werden müssten. Bei diesem Kolben findet auch während dessen Verlagerung keine Beschichtung der inneren Mantelfläche mit einem Gleitmittel statt. Es wird also gänzlich auf ein Gleitmittel verzichtet.

Dabei ist es möglich, dass das glatte, unpolare Material PTFE umfasst, vorzugsweise aus PTFE besteht. Dieses unter dem Handelsnamen Teflon bekannte Material weist besonders gute Gleiteigenschaften auf und steht darüber hinaus kostengünstig zur Verfügung.

Aufgabe der Erfindung ist es außerdem, einen medizinischen Hohlkörper zu schaffen, bei dem eine Beeinträchtigung einer in dem Hohlkörper vorhandene pharmazeutische Substanz während ihrer Lagerung mit hoher Sicherheit vermieden wird.

Die Aufgabe wird gelöst durch einen medizinischen Hohlkörper, der die Merkmale des Anspruchs 11 aufweist.

Der medizinische Hohlkörper, insbesondere eine Spritze oder Karpule, umfasst mindestens einen Kolben, der in einem Innenraum des Hohlkörpers entlang dessen Längsachse verlagerbar ist. Er zeichnet sich dadurch aus, dass der Kolben gemäß einem der Ansprüche 1 bis 10 ausgebildet ist. Der Kolben weist also eine Sperreinrichtung der oben genannten Art auf.

Die Erfindung wird im Folgenden anhand der Zeichnung näher erläutert. Es zeigen:
- Figur 1: eine schematische Ansicht eines nicht zur Erfindung gehörigen medizinischen Hohlkörpers mit einem einem Kolben;
- Figur 2: eine weitere schematische Ansicht des medizinischen Hohlkörpers gemäß Figur 1 während einer Verlagerung des Kolbens;
- Figur 3: eine schematische Ansicht eines weiteren nicht zur Erfindung gehörigen medizinischen Hohlkörpers mit einem abgewandelten Kolben;
- Figur 4: eine schematische Ansicht eines ersten Ausführungsbeispiels eines Kolbens in einer ersten Funktionsstellung;
- Figur 5: den Kolben gemäß Figur 4 in einer zweiten Funktionsstellung;
- Figur 6: den Kolben gemäß Figur 4 in einer dritten Funktionsstellung;
- Figur 7: eine schematische Darstellung eines zweiten Ausführungsbeispiels eines Kolbens;
- Figur 8: eine schematische Darstellung eines dritten Ausführungsbeispiels eines Kolbens, und
- Figur 9: eine schematische Darstellung eines nicht zur Erfindung gehörigen Kolbens.

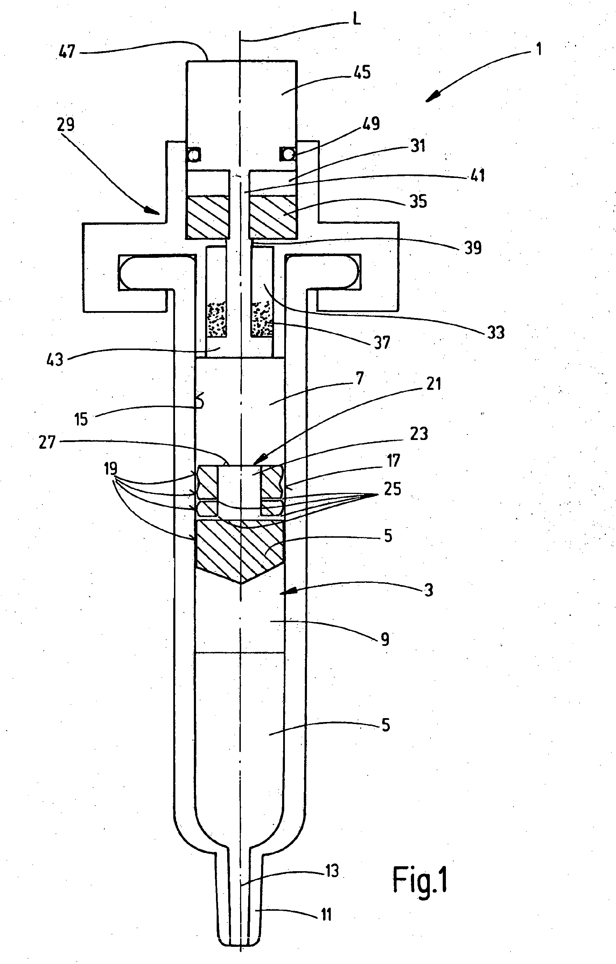

Figur 1 zeigt einen nicht zur Erfindung gehörenden medizinischen Hohlkörper 1, der hier als Spritze ausgebildet ist. In anderen, nicht dargestellten Fällen kann der Hohlkörper 1 auch als Karpule, Doppel- oder Mehrkammerspritze oder -karpule, Pen, Autoinjektor oder in sonstiger geeigneter Weise ausgebildet sein. Der Hohlkörper 1 ist im Wesentlichen zylindrisch ausgebildet und umfasst einen Innenraum 3, in dem ein ebenfalls im Wesentlichen zylindrischer Kolben 5 entlang der aufeinanderfallenden Längsachsen L des Kolbens 5 und des Hohlkörpers 1 verlagerbar ist. Der Kolben 5 trennt den Innenraum 3 in einen oberen Teilraum 7 und einen unteren Teilraum 9. In dem unteren Teilraum 9 ist vorzugsweise eine pharmazeutische Substanz S angeordnet, die durch einen in einem Ansatzstück 11 gebildeten Kanal 13, der in Fluidverbindung mit dem unteren Teilraum 9 steht, abgebbar ist, wenn der Kolben 5 entlang der Längsachse L nach unten, also in Richtung auf das Ansatzstück 11, verlagert wird.

Den Innenraum 3 umschließt eine im Wesentlichen zylindrische innere Mantelfläche 15 des Hohlkörpers 1. Der Kolben 5 weist eine ebenfalls im Wesentlichen zylindrische äußere Mantelfläche 17 auf, die bei dem dargestellten Ausführungsbeispiel - im Querschnitt gesehen - quasi wellenförmig ausgebildet ist, sodass sich - in axialer Richtung gesehen - Bereiche größeren Durchmessers mit Bereichen kleineren Durchmessers abwechseln. Vorzugsweise umfasst der Kolben 5 zumindest im Bereich der äußeren Mantelfläche 17 ein Material, welches elastischer ist als das Material, aus dem zumindest der Bereich des Hohlkörpers 1 besteht, der die innere Mantelfläche 15 umfasst. Bevorzugt sind dann die Bereiche größeren Durchmessers der Mantelfläche 17 so ausgebildet, dass ihr Durchmesser geringfügig größer ist als der Innendruchmesser des Innenraums 3. Wird in diesem Fall der Kolben 5 in den Innenraum 3 eingebracht, werden die Bereiche größeren Durchmessers der äußeren Mantelfläche 17 komprimiert, sodass sich hier Gleitflächen 19 bilden, mit denen der Kolben 5 an dem Hohlkörper 1 anliegt. Bei einem anderen, nicht dargestellten Ausführungsbeispiel kann die äußere Mantelfläche 17 eine Zylinderfläche sein, wobei bevorzugt dann der Außendurchmesser dieses Zylinders geringfügig größer ist als der Innendurchmesser des Innenraums 3. In diesem Fall bildet die gesamte äußere Mantelfläche 17 des Kolbens 5 eine einzige Gleitfläche 19.

Bei einer Verlagerung des Kolbens 5 in dem Hohlkörper 1 gleitet die mindestens eine Gleitfläche 19 an der inneren Mantelfläche 15 des Innenraums 3.

Der Kolben 5 weist einen Aufnahmebereich 21 auf, der ein Gleitmittel umfasst. Bei anderen, nicht dargestellten Kolben kann mehr als ein Aufnahmebereich 21 vorgesehen sein. Beispielsweise kann der Aufnahmebereich 21 unterteilt sein, sodass mehrere Aufnahmebereiche entstehen. Es können auch separate Aufnahmebereiche 21 von dem Kolben 5 umfasst sein.

Der Aufnahmebereich 21 umfasst hier einen Hohlraum 23, der als Vorratsbehälter für das Gleitmittel dient.

Als Gleitmittel kann vorzugsweise Silikon, ein Silikonöl oder eine Silikonölemulsion verwendet werden. Auch ein Gemisch dieser Stoffe ist möglich. Sind verschiedene Aufnahmebereiche vorgesehen, können diese bevorzugt auch verschiedene Gleitmittel aufnehmen, sodass hier eine große Variation möglich ist.

Es ist mindestens ein Abgabemittel vorgesehen, durch das das mindestens eine Gleitmittel von dem mindestens einen Aufnahmebereich 21 an die mindestens eine Gleitfläche 19 abgebbar ist. Das Abgabemittel umfasst hier Kanäle 25, die den Hohlraum 23 mit der äußeren Mantelfläche 17 verbinden und aus dieser austreten, sodass sie insbesondere von dem Aufnahmebereich 21 zu den Gleitflächen 19 führen. Dabei kann in den Kanälen 25 Gleitmittel strömen. Bei einem anderen, nicht dargestellten Kolbens kann auch ein einziger Kanal 25 vorgesehen sein. Bevorzugt wird aber, wenn mehrere Kanäle 25 die Mantelfläche 17 entlang ihres Umfangs vorzugsweise in gleicher Winkelteilung schneiden, sodass das Gleitmittel - in Umfangsrichtung gesehen - sehr gleichmäßig auf den Gleitflächen 19 verteil werden kann. In dem dargestellten Beispiel sind in axialer Richtung zueinander versetzt zwei ringförmige Bereiche der Mantelfläche 17 vorgesehen, die von Kanälen 25 geschnitten werden. Je nach axialer Längenausdehnung des Kolbens 5 können mehr oder weniger solcher vorzugsweise ringförmigen Bereiche vorgesehen sein, wodurch das Gleitmittel auch in axialer Richtung gleichmäßig auf die eine Gleitfläche 19 oder die verschiedenen Gleitflächen 19 verteilt werden kann. Selbstverständlich ist bei anderen, nicht dargestellten Ausgestaltungen auch eine weniger symmetrische Verteilung von Kanälen 25 möglich.

In Figur 1 ist der Kolben 5 in einem ersten Zustand dargestellt, in dem kein Gleitmittel aus dem mindestens einen Aufnahmebereich abgebbar ist. Der Hohlraum 23 durchsetzt hier nicht den gesamten Kolben 5 entlang dessen axialer Erstreckung, sondern erstreckt sich ausgehend von dem dem Ansatzstück 11 - in axialer Richtung gesehen - gegenüberliegenden Ende des Kolbens 5 nur etwa bis zu dessen Mitte. Das dem Anschlussstück 11 - in axialer Richtung gesehen - gegenüberliegende Ende des Hohlraums 23 ist hier durch eine vorzugsweise flüssigkeitsdichte Membran 27 verschlossen, die verhindert, dass in diesem Bereich Gleitmittel austreten kann. In dem dargestellten Zustand des Kolbens kann auch kein Gleitmittel aus den Kanälen 25 austreten, weil diese in sehr kleine, zwischen den Gleitflächen 19 angeordnete Bereiche münden, die nur ein minimales Volumen an Gleitmittel aufnehmen können. Wesentlich ist aber, dass in dem dargestellten ersten Zustand der Kolben 5 und insbesondere der Hohlraum 23 drucklos ist, sodass sich im oberen Bereich des Hohlraums 23 ein Unterdruck einstellen würde, wenn Gleitmittel aus den Kanälen 25 austreten würde. Insgesamt zeigt sich also, dass in diesem Zustand kein Gleitmittel aus dem mindestens einen Aufnahmebereich 21 abgebbar ist.

Die Membran 27 verschließt den Hohlraum 23, also auch den Aufnahmebereich 21 in dessen oberem Bereich. Sind mehrere Aufnahmebereiche 21 vorgesehen, ist es möglich, diese durch mehrere Membranen zumindest bereichsweise zu verschließen, vorzugsweise so, dass jedem Aufnahmebereich 21 eine Membran zugeordnet ist. In anderen Ausführungsbeispielen können auch einem Aufnahmebereich mehrere Membranen zugeordnet sein, die diesen bereichsweise verschließen. Wesentlich ist, dass der Kolben 5 mindestens eine Membran 27 umfasst, die den mindestens einen Aufnahmebereich 21 zumindest bereichsweise verschließt.

Der Kolben 5 umfasst in einem bevorzugten Ausführungsbeispiel ein Elastomer. Besonders bevorzugt umfasst der Kolben 5 das Elastomer im Bereich seiner äußeren Mantelfläche 17 und besonders im Bereich der mindestens einen Gleitfläche 19. So kann die Elastizität des Kolbens 5 in diesem Bereich sichergestellt werden, die eine dichte Anlage der mindestens einen Gleitfläche 19 an dem Hohlkörper 1 ermöglicht. Der Kolben 5 kann vorzugsweise auch aus einem Elastomer bestehen. Er ist dann einfacher herstellbar als dies der Fall ist, wenn er verschiedene Materialien umfasst und beispielsweise nur in äußeren Bereichen ein Elastomer aufweist.

Um die Haftreibung beziehungsweise auch die Gleitreibung des Kolbens 5 über die durch das Gleitmittel erzielte Wirkung hinaus zu verringern, kann der Kolben 5 vorzugsweise PTFE umfassen, besonders bevorzugt aus PTFE bestehen. Umfasst der Kolben 5 nur bereichsweise PTFE, ist dieses bevorzugt im Bereich der äußeren Mantelfläche 17 und insbesondere im Bereich der mindestens einen Gleitfläche 19 vorgesehen. Eine Ausstattung des Kolbens 5 mit PTFE zumindest im Bereich der mindestens einen Gleitfläche 19 führt dazu, dass die Haft- beziehungsweise Gleitreibung bereits ohne zusätzliche Schmierung deutlich herabgesetzt wird. Zusammen mit dem Gleitmittel ergibt sich so eine besonders wenig kraftaufwendige Verlagerbarkeit des Kolbens 5.

In dem dargestellten Beispiel umfasst der medizinische Hohlkörper 1 eine Vorrichtung 29, mittels derer Druckkräfte auf den Kolben 5 durch eine chemische Reaktion ausübbar sind. Hierzu umfasst die Vorrichtung 29 eine erste Kammer 31 und eine zweite Kammer 33. In den Kammern 31, 33 sind Substanzen angeordnet, die, solange sie räumlich voneinander getrennt sind, zumindest über eine typische Lagerzeit eines vorgefüllten Hohlkörpers 1 stabil sind. Werden sie jedoch miteinander in Verbindung gebracht, können sie - gegebenenfalls nach Überwindung einer Aktivierungsbarriere - miteinander eine chemische Reaktion eingehen, bei der mindestens ein Gas freigesetzt wird. In der ersten Kammer 31 liegt eine Substanz 35 und in der zweiten Kammer 33 eine Substanz 37 vor. Die zweite Substanz 37 kann beispielsweise Natriumhydrogencarbonat umfassen, wobei die erste Substanz 35 vorzugsweise eine organische Säure, beispielsweise Zitronensäure, oder eine Mineralsäure umfasst. Im Falle einer Reaktion der Säure mit dem Natriumhydrogencarbonat wird Kohlendioxid freigesetzt, sodass ein Gasdruck aufbaubar ist. Prinzipiell reicht es, wenn in einer der Kammern 31, 33 ein metastabiler, bei Überwindung einer Aktivierungsbarriere zersetzbarer Reinstoff vorliegt, der bei seiner Zersetzung ein Gas freisetzt. In der anderen Kammer kann dann ein Katalysator angeordnet sein, der zur Auslösung der chemischen Reaktion mit dem Reinstoff in Kontakt gebracht wird. Bei wieder einem anderen Ausführungsbeispiel ist es auch möglich, dass die beiden Substanzen 35, 37 erst nach Überwindung einer Aktivierungsbarriere miteinander reagieren.

Hierzu kann in einer der beiden Kammern 31, 33 zusätzlich zu einer der beiden Substanzen 35, 37 ein Katalysator angeordnet sein, der seine Wirkung erst entfaltet, wenn die beiden Substanzen 35, 37 in Kontakt gebracht werden. Bei einer anderen Ausgestaltung kann auch ein Startmechanismus in die Vorrichtung 29 integriert sein, der die Aktivierungsbarriere thermisch, elektrochemisch oder auf andere an sich bekannte und geeignete Weise überwindet. Hierzu können beispielsweise Heizvorrichtungen oder Elektroden vorgesehen sein. Wird die Aktivierungsbarriere eines metastabilen Reinstoffs thermisch, elektrochemisch oder auf andere, physikalische Weise überwunden, genügt es, eine einzige Kammer vorzusehen, die den Reinstoff umfasst, außerdem einen geeigneten Startmechanismus für dessen Zersetzung.

Sind zwei Substanzen 35, 37 vorgesehen, die miteinander unter Gasentwicklung reagieren, wenn sie in Kontakt gebracht werden, müssen diese vor einer Betätigung der Vorrichtung 29 voneinander getrennt in den Kammern 31, 33 aufbewahrt werden. Hierzu ist ein Trennelement 39 vorgesehen, das einer als Teil einer Stange 41 eines Verschlusselements 43 ausgebildet ist.

Bei anderen, nicht dargestellten Ausgestaltungen kann das Trennelement als durchstechbares Septum, als zerreiß- oder zerbrechbare Membran, als massives Zerbrechelement oder in anderer geeigneter Weise ausgebildet sein.

Das Verschlusselement 43 verschließt die zweite Kammer 33 dicht gegenüber dem oberen Teilraum 7, sodass die Substanz 37 nicht in diesen austreten kann. Das Trennelement 39 trennt die Kammern 31, 33 voneinander, sodass die Substanzen 35, 37 nicht in Kontakt miteinander kommen können. Es ist ein Betätigungselement 45 vorgesehen, das dazu dient, die Kammern 31, 33 in Fluidverbindung miteinander zu bringen, sodass die Substanzen 35 und 37 in Kontakt kommen und miteinander reagieren können. Je nach Ausführungsbeispiel des Trennelements 39 kann das Betätigungselement 45 als Hohlnadel, massiver Stößel oder in anderer geeigneter Weise ausgebildet sein. Bei dem dargestellten Ausführungsbeispiel weist das Betätigungselement 45 an seinem oberen Ende einen Betätigungsbereich 47 auf, in den in axialer Richtung wirkende Druckkräfte einleitbar sind. Es umfasst auch eine Dichtung 49, die hier als in einer Ringnut angeordneter O-Ring ausgebildet ist. Das Betätigungselement 45 ist mit der Stange 41 und damit auch mit dem Verschlusselement 43 und dem Trennelement 39 verbunden, sodass sich diese Elemente bei Einleitung axialer Druckkräfte in den Betätigungsbereich 47 gemeinsam nach unten, also in Richtung des Ansatzstücks 11, verlagern.

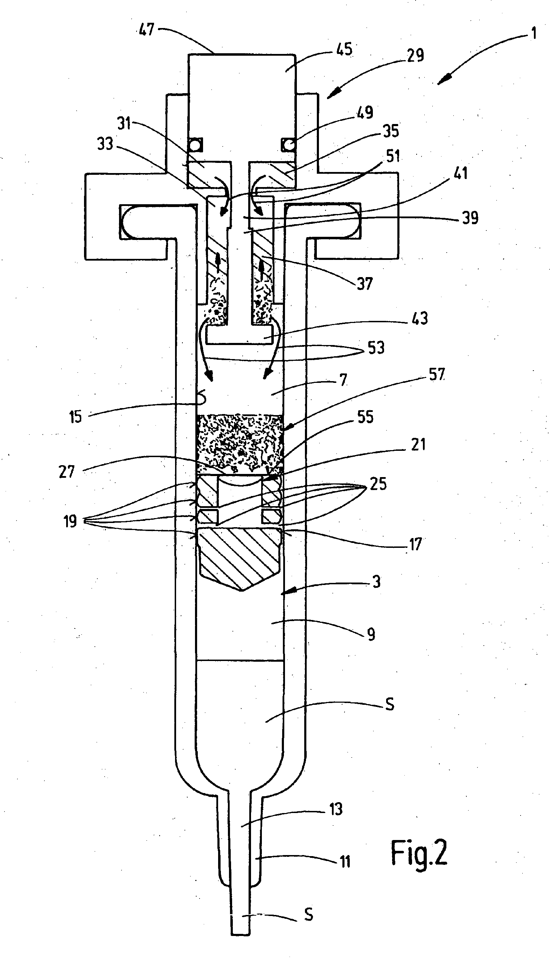

Figur 2 zeigt den Hohlkörper gemäß Figur 1 bei aktivierter Vorrichtung 29 und insbesondere während einer Verlagerung des Kolbens 5. Gleiche und funktionsgleiche Elemente sind mit gleichen Bezugszeichen versehen, sodass insofern auf die vorangegangene Beschreibung verwiesen wird. In Figur 2 wird deutlich, dass durch die Verlagerung des Betätigungselements 45 und der damit verbundenen Elemente nach unten zum einen eine Fluidverbindung zwischen den Kammern 31, 33 und zum anderen eine Fluidverbindung zwischen der Kammer 33 und dem oberen Teilraum 7 geschaffen wird. Hierdurch tritt die Substanz 35 entlang der Pfeile 51 von der ersten Kammer 31 in die zweite Kammer 33 ein. Dort kommt sie in Kontakt mit der Substanz 37, sodass sie mit dieser reagieren kann. Gleichzeitig tritt die Substanz 37 beziehungsweise ein Gemisch der Substanzen 35, 37 entlang der Pfeile 53 aus der zweiten Kammer 37 aus und gelangt in den oberen Teilraum 7. Bei der Reaktion der Substanzen 35, 37 wird mindestens ein Gas freigesetzt, dem ein begrenztes Volumen zur Verfügung steht, das durch die Kammern 31, 33 und den oberen Teilraum 7 definiert ist.

Bei dem mindestens einen freigesetzten Gas handelt es sich bevorzugt um ein chemisch nicht aggressives, nichttoxisches Gas, besonders bevorzugt um ein Inertgas. Die fortgesetzte Gasbildung während der Reaktion führt zu einem Anstieg des Drucks in dem dem Gas zur Verfügung stehenden Volumen, wobei der Druck auf eine dem oberen Teilraum 7 zugewandte Oberfläche 55 des Kolbens 5 und insbesondere auch auf die Membran 27 wirkt. Bei einem nicht dargestellten Hohlkörper kann die Membran 27 elastisch ausgebildet sein, sodass sie durch die auf sie wirkenden Druckkräfte in den Hohlraum 23 hineingewölbt wird. Dadurch werden die Druckkräfte an das in dem Aufnahmebereich 21 angeordnete Gleitmittel weitergegeben, welches über die Kanäle 25, die als Abgabemittel wirken, an die Gleitfläche 19 abgegeben wird. Auf diese Weise bildet sich ein Schmierfilm zwischen der mindestens einen Gleitfläche 19 und der inneren Mantelfläche 15 aus, wodurch die dort herrschenden Reibungskräfte herabgesetzt werden und der Kolben 5 leichter verlagerbar ist. Die auf die Oberfläche 55 wirkenden Druckkräfte führen zugleich auch zu einer Verlagerung des Kolbens 5 nach unten auf das Ansatzstück 11 hin, sodass sich der Teilraum 7 vergrößert, während sich der Teilraum 9 verkleinert. Hierdurch wird die in dem unteren Teilraum 9 angeordnete Substanz S durch den Kanal 13 in dem Ansatzstück 11 ausgetrieben, was hier schematisch dargestellt ist. Die Substanz S kann also dem Hohlkörper 1 entnommen werden, vorzugsweise kann eine Injektion stattfinden, wenn der Hohlkörper als Spritze, Karpule oder sonstige Injektionsvorrichtung ausgebildet ist, wobei eine nicht dargestellte Injektionsnadel mit dem Ansatzstück 11 gekoppelt ist.

Bei dem dargestellten Hohlkörper ist die Membran 27 allerdings nicht elastisch, sondern für Gase durchlässig ausgebildet, sodass das während der Reaktion freigesetzte mindestens eine Gas durch die Membran 27 permeieren und in den Hohlraum 23 eintreten kann. Es findet also ein Druckausgleich zwischen dem oberen Teilraum 7 und dem Hohlraum 23 statt, weshalb sich die Membran 27 nicht verformt. Gleichzeitig werden die in dem Teilraum 7 wirkenden Druckkräfte an das in dem Aufnahmebereich 21 angeordnete Gleitmittel weitergegeben, sodass dieses über die Kanäle 25 an die mindestens eine Gleitfläche 19 abgegeben werden kann. Durch die auf die Oberfläche 55 wirkenden Druckkräfte wird außerdem der Kolben 5 nach unten verlagert. Dabei entsteht an der inneren Mantelfläche 15 ein hinter dem Kolben 5 zurückbleibender Schmierfilm, der hier als gepunkteter Bereich 57 dargestellt ist. Das Gleitmittel haftet also zumindest teilweise nach einer Verlagerung des Kolbens 5 an der inneren Mantelfläche 15.

In einer anderen, nicht dargestellten Ausgestaltung kann der Kolben 5 auch aus einem ausquetsch- beziehungsweise ausdrückbaren Material bestehen, das vorzugsweise eine Anzahl Poren aufweist, in denen das Gleitmittel angeordnet ist. Die Poren bilden also Aufnahmebereiche, in denen das Gleitmittel aufnehmbar ist. Werden Druckkräfte in den Kolben 5 eingeleitet, verformt sich dieser zumindest bereichsweise, was zu einer Verkleinerung des Volumens der einzelnen Poren, also der Aufnahmebereiche, führt. Hierdurch ist das Gleitmittel aus den Poren abgebbar, sodass die an der Mantelfläche 17 des Kolbens angeordneten Poren in Verbindung mit den die äußeren mit den inneren Poren und diese Poren untereinander verbindenden Kanälen auch als Abgabemittel wirken, wobei das Gleitmittel hier von den Aufnahmebereichen an die mindestens eine Gleitfläche abgebbar ist. Der Kolben 5 wird in diesem Fall also von den Druckkräften quasi ausgedrückt oder ausgequetscht, wobei Gleitmittel austritt, und wobei der Kolben 5 gleichzeitig durch die auf seine Oberfläche 55 wirkenden Druckkräfte verlagert wird.

Insgesamt wird deutlich, dass der Kolben in Figur 2 in einem zweiten Zustand dargestellt ist, in dem Gleitmittel durch das mindestens eine Abgabemittel an die mindestens eine Gleitfläche abgebbar ist. In dem dargestellten Ausführungsbeispiel ist der Kolben 5 in dem zweiten Zustand mit einem Druck beaufschlagt, wobei die Druckkräfte an das Gleitmittel weitergegeben werden und dieses aus dem Aufnahmebereich austreiben, sodass es an die mindestens eine Gleitfläche 19 abgegeben werden kann.

Wie bereits gesagt wurde, kann die Membran 27 elastisch aber auch für Gase durchlässig ausgebildet sein. Selbstverständlich ist es bei anderen bevorzugten Ausführungsbeispielen auch möglich, die Membran 27 sowohl elastisch als auch für Gase durchlässig auszubilden.

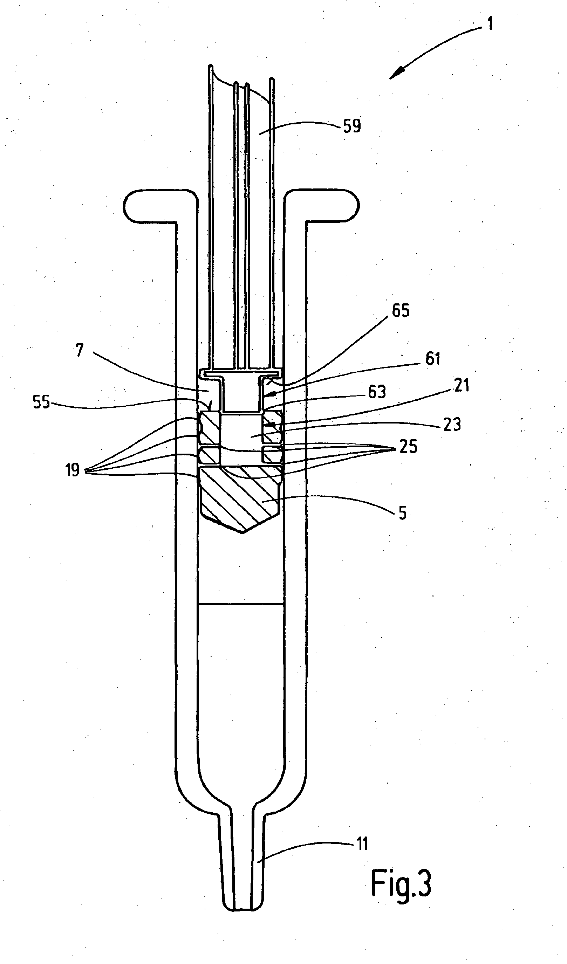

Figur 3 zeigt eine abweichende Ausgestaltung eines ebenfalls nicht zur Erfindung gehörenden Hohlkörpers 1, in dem ein weiterer abgewandelter Kolben 5 angeordnet ist. Gleiche und funktionsgleiche Elemente sind mit gleichen Bezugszeichen versehen, sodass insofern auf die vorangegangene Beschreibung verwiesen wird. Im Unterschied zu dem in den Figuren 1 und 2 dargestellten Fällen eines medizinischen Hohlkörpers 1 weist der medizinische Hohlkörper 1 gemäß Figur 3 eine Kolbenstange 59 auf, die mit dem Kolben 5 verbunden ist. Hierzu umfasst die Kolbenstange 59 einen Verbindungsbereich 61, der so ausgebildet ist, dass er in den Hohlraum 23 des Kolbens 5 einbringbar ist. Der Verbindungsbereich 61 umfasst vorzugsweise an seinem unteren Ende eine Dichtungseinrichtung 63, die bevorzugt als in Umfangsrichtung umlaufender radialer Vorsprung ausgebildet ist und den Hohlraum 23 gegenüber dem oberen Teilraum 7 dicht verschließt.

Mittels der Kolbenstange 59 sind Druckkräfte auf den Kolben 5 ausübbar. In Figur 3 ist ein Zustand des Kolbens 5 dargestellt, in welchem dieser nicht mit einem Druck beaufschlagt ist. Dies entspricht dem ersten Zustand des Kolbens 5, in dem kein Gleitmittel aus dem mindestens einen Aufnahmebereich 21, der hier durch den Hohlraum 23 gebildet wird, abgebbar ist. Der Kolben 5 kann in seinem zweiten Zustand, in dem Gleitmittel durch die als Abgabemittel wirkenden Kanäle 25 an die mindestens eine Gleitfläche 19 abgebbar ist, versetzt werden, in dem vermittelt durch die Kolbenstange 59 Druckkräfte in den Kolben 5 eingeleitet werden. Die Kolbenstange 59 wird dabei in Richtung auf das Ansatzstück 11 hin, also nach unten verlagert. Im Unterschied zu dem in den Figuren 1 und 2 dargestellten Kolbens 5 weist der in Figur 3 dargetellte keine Membran 27 auf, sondern der Hohlraum 23 wird gegenüber dem oberen Teilraum 7 ausschließlich durch die Dichtungseinrichtung 63 verschlossen. Wird also die Kolbenstange 59 nach unten verlagert, dringt zunächst der Verbindungsbereich 61 mit der Dichtungseinrichtung 63 in den Hohlraum 23 ein und beaufschlagt so das in dem Aufnahmebereich 21 angeordnete Gleitmittel mit einem Druck. Durch diesen wird das Gleitmittel über die Kanäle 25 aus dem Hohlraum 23 ausgetrieben und an die mindestens eine Gleitfläche 19 abgegeben. Je nach den herrschenden Kräfteverhältnissen kann der in dem Hohlraum 23 herrschende Druck ausreichen, um auch eine Verlagerungsbewegung des Kolbens 5 in Richtung auf das Ansatzstück 11 hin, also nach unten, zu bewirken. In diesem Fall wird durch die in den Hohlraum 23 und damit den Kolben 5 eingeleitete Druckkraft sowohl ein Austreiben des Gleitmittels aus dem mindestens einen Aufnahmebereich 21 als auch eine Verlagerung des Kolbens 5 bewirkt.

Reicht die in dem Hohlraum 23 herrschende Druckkraft nicht aus, eine Verlagerung des Kolbens 5 zu bewirken, wird zuerst das Gleitmittel vollständig ausgetrieben, während der Verbindungsbereich 61 sich in den Hohlraum 23 hineinverlagert. Wenn der Verbindungsbereich 61 vollständig in dem Hohlraum 23 angeordnet ist, schlägt die Kolbenstange 59 mit einer vorzugsweise als Ringschulter ausgebildeten Anlagefläche 65 an der Oberfläche 55 des Kolbens 5 an. In diesem Fall kann über die Anlagefläche 65 eine Kraft in die Oberfläche 55 eingeleitet werden, die dann eine Verlagerung des Kolbens 5 bewirkt.

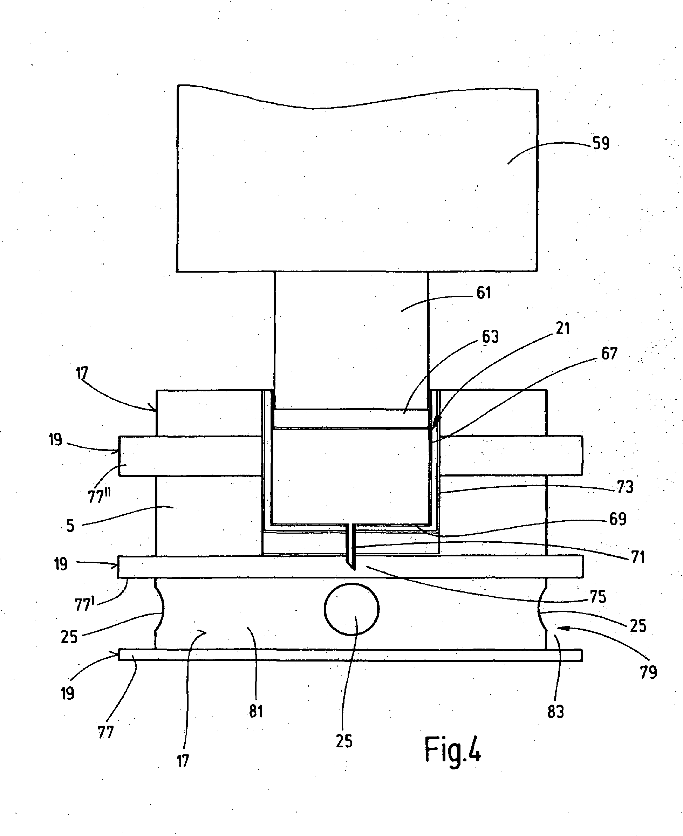

Figur 4 zeigt ein erstes Ausführungsbeispiel eines Kolbens 5. Gleiche und funktionsgleiche Elemente sind mit gleichen Bezugszeichen versehen, sodass insofern auf die vorangegangene Beschreibung verwiesen wird. Bei dem dargestellten Ausführungsbeispiel umfasst der mindestens eine Aufnahmebereich 21 mindestens ein Reservoir 67. Dieses ist hier als ein im Wesentlichen zylindrisches Gefäß ausgebildet, das einen Boden 69 umfasst, der ein Abgabeelement aufweist, das hier als Nadel 71 ausgebildet ist.

Bei einem anderen, nicht dargestellten Ausführungsbeispiel, kann das Reservoir mindestens einen Mikroballon umfassen, wobei ein solcher Mikroballon eine vorzugsweise elastische Hülle aufweist, die ein Gleitmittelvolumen umschließt. Diese Hülle ist zerreißbar ausgebildet und kann insbesondere bei Einleitung von Druckkräften platzen und dadurch das von ihr umschlossene Gleitmittel freisetzen. Es ist auch ein Ausführungsbeispiel möglich, bei dem anstelle eines Reservoirs mindestens ein Mikroballon vorgesehen ist. Selbstverständlich ist es auch möglich, mehr als einen Mikroballon in Zusammenhang mit einem Reservoir oder ohne ein Reservoir einzusetzen. In letztem Fall definiert der mindestens eine Mikroballon den Aufnahmebereich 21.

In dem dargestellten Ausführungsbeispiel wird das Reservoir 67 durch eine Dichtungseinrichtung 63 einer Kolbenstange 59 nach oben dicht verschlossen. Bei einem bevorzugten, nicht dargestellten Ausführungsbeispiel ist es möglich, das Reservoir 67 durch eine elastische und/oder für Gase durchlässige Membran 27 zu verschließen.

Das Reservoir 67 ist in axialer Richtung verlagerbar in einer Ausnehmung 73 des Kolbens 5 angeordnet. Der Begriff "axiale Richtung" spricht hier die Richtung an, die durch die nicht dargestellte Längsachse des Kolbens 5 definiert ist, welche mit der ebenfalls nicht dargestellten Längsachse eines medizinischen Hohlkörpers 1, mit dem der Kolben 5 zusammenwirkt, zusammenfällt.

In dem dargestellten Zustand des Kolbens 5 ist das Reservoir 67 in einer ersten, oberen Stellung angeordnet, in der die Nadel 71 einen Dichtungsbereich 75 nicht durchdringt, sodass kein Gleitmittel aus dem Reservoir 67 nach unten durch die als Abgabeelement ausgebildete Nadel 71 abgebbar ist. In diesem ersten Zustand des Kolbens 5 ist dieser vorzugsweise drucklos.

An seiner äußeren Mantelfläche 17 weist der dargestellte Kolben 5 mehrere, entlang seiner Umfangsrichtung umlaufende, radiale Vorsprünge auf, deren Außendurchmesser in bereits beschriebener Weise auf den Innendurchmesser eines nicht dargestellten medizinischen Hohlkörpers 1 abgestimmt sind, sodass sich eine Dichtwirkung ergibt und insbesondere mindestens eine Gleitfläche 19 - hier drei Gleitflächen 19 - zur Anlage an dem Hohlkörper 1 ausgebildet werden. Ein erster, unterer Vorsprung 77 ist - in axialer Richtung gesehen - relativ kurz beziehungsweise dünn, insbesondere quasi lamellenartig ausgebildet, und dient im Wesentlichen dazu, einen Bereich 79, in den in einem zweiten, nicht dargestellten Zustand des Kolbens 5 Gleitmittel eingebracht wird, gegenüber dem unteren Teilraum 9 des medizinischen Hohlkörpers 1 abzudichten, sodass das Gleitmittel nicht in Kontakt mit der Substanz S kommen kann. Insbesondere die - in axialer Richtung gesehen - relativ schmale, lamellenartige Ausgestaltung des Vorsprungs 77 dient auch dazu, die dort im Bereich der mindestens einen Gleitfläche 19 wirkende Reibung zu minimieren. Bei dem dargestellten Ausführungsbeispiel sind zwei weitere Vorsprünge 77', 77" vorgesehen, die - ebenfalls in axialer Richtung gesehen - breiter ausgebildet sind als der erste Vorsprung 77. Insbesondere diese Vorsprünge 77' und 77" dienen zur Führung und Stabilisierung des Kolbens 5 in dem Innenraum 3 des Hohlkörpers 1, wobei sie jeweils eine Gleitfläche 19 zur Anlage an dem Hohlkörper 1 umfassen.

Die restliche äußere Mantelfläche 17 weist einen kleineren Durchmesser auf als im Bereich der Vorsprünge 77, 77', 77". Der Bereich 79 ist zwischen dem Vorsprung 77 und dem Vorsprung 77' vorgesehen. Er umfasst einen Kernbereich 81 des Kolbens 5, der von einem relativ zu den Gleitflächen 19 der Vorsprünge 77, 77' zurückspringenden Teil der Mantelfläche 17 umschlossen wird. Durch den radialen Versatz der Mantelfläche 17 ergibt sich ein Ringraum 83, der den Kernbereich 81 - in Umfangsrichtung gesehen - ringförmig umgreift.

Der Kernbereich 81 wird durch mindestens einen Kanal 25 durchsetzt, der einen nicht dargestellten, hohlen Mittelbereich des Kernbereichs 81 mit dem Bereich 79, also insbesondere dem Ringraum 83, verbindet. Der hohle Mittelbereich ist unterhalb des Dichtungsbereichs 75 angeordnet, der durch die Nadel 71 durchstechbar ist. Bei dem vorliegenden Ausführungsbeispiel sind vier Kanäle 25 vorgesehen, die - in Umfangsrichtung gesehen - in gleicher Winkelteilung in den Ringraum 83 münden.

Es wird deutlich, dass der Kolben 5 in Figur 4 in einer ersten Funktionsstellung dargestellt ist, die dem ersten Zustand entspricht, in welchem kein Gleitmittel aus dem mindestens einen Aufnahmebereich 21, hier also dem Reservoir 67, abgebbar ist, weil die als Abgabeelement dienende Nadel 71 durch den Dichtungsbereich 75 verschlossen ist.

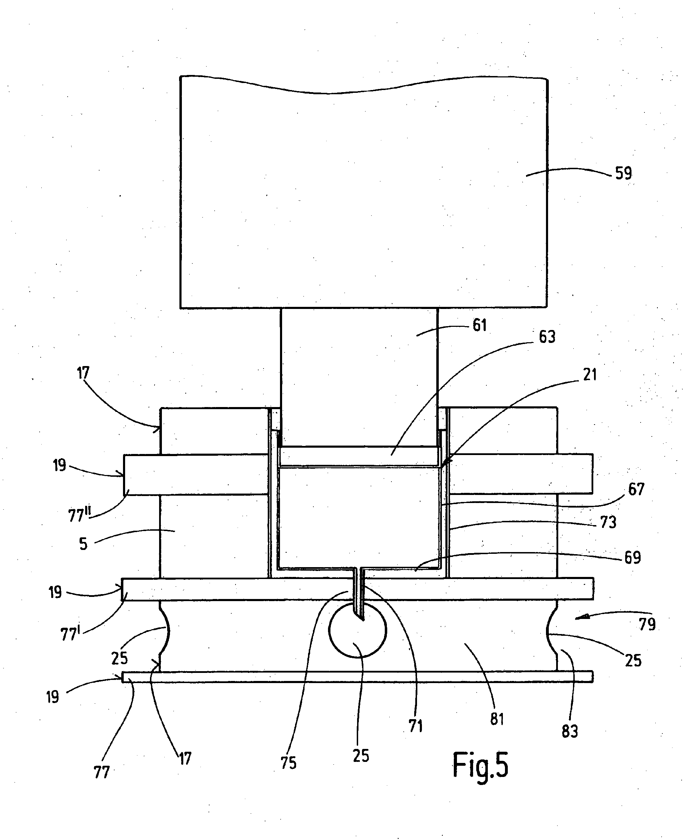

Figur 5 zeigt den Kolben 5 in einer zweiten Funktionsstellung, die dem zweiten Zustand entspricht, in dem Gleitmittel aus dem Aufnahmebereich 21, hier also dem Reservoir 67, an die mindestens eine Gleitfläche 19 abgebbar ist. Gleiche und funktionsgleiche Elemente sind mit gleichen Bezugszeichen versehen, sodass insofern auf die vorangegangene Beschreibung verwiesen wird. Es ist offensichtlich, dass der Kolben 5 in Figur 4 in einem im Wesentlichen drucklosen Zustand vorliegt. Wird über die Kolbenstange 59 ein Druck in das Reservoir 67 eingeleitet, wobei das dort angeordnete Gleitmittel mit dem Druck beaufschlagt wird, verlagert sich zunächst das Reservoir 67 nach unten, sodass die Nadel 71 den Dichtungsbereich 75 durchdringt. In Figur 5 ist nun der Zustand dargestellt, in dem die Nadel 71 den Dichtungsbereich 75 gerade durchstochen hat. Es ist damit eine Fluidverbindung zwischen dem Reservoir 67 und dem nicht dargestellten, hohlen Mittelbereich des Kernbereichs 81 hergestellt.

Es wird nun zunächst Folgendes deutlich: Der Kolben 5 umfasst bevorzugt eine Sperreinrichtung, durch die der mindestens eine Kanal 25 in dem ersten Zustand des Kolbens 5 sperrbar und in dem zweiten Zustand des Kolbens 5 freigebbar ist. In dem vorliegenden Ausführungsbeispiel umfasst die Sperreinrichtung die Nadel 71 und einen mit der Nadel 71 durchstechbaren Bereich, nämlich den Dichtungsbereich 75. In dem dargestellten zweiten Zustand des Kolbens 5, in dem die Nadel 71 den Dichtungsbereich 75 durchsticht, ist eine Fluidverbindung zwischen dem Reservoir 67 und dem mindestens einen Kanal 25 hergestellt, der Kanal 25 ist also freigegeben.

Bei anderen, nicht dargestellten Ausführungsbeispielen kann die Sperreinrichtung auch eine Sollbruchstelle, eine zerreißbare Membran und/oder eine in Sperrrichtung vorgespannte Lippendichtung umfassen. Die Formulierung "Sperrrichtung" spricht hier an, dass der mindestens eine Kanal 25 gesperrt ist, wenn die Lippendichtung in diese Richtung verlagert ist. Die Vorspannung der Lippendichtung in Sperrrichtung stellt sicher, dass keine Fluidverbindung zwischen dem mindestens einen Aufnahmebereich und dem mindestens einen Kanal 25 vorliegt, wenn sich der Kolben 5 in seinem ersten Zustand befindet, also vorzugsweise drucklos ist. In dem zweiten Zustand des Kolbens 5, in dem dieser vorzugsweise mit einem Druck beaufschlagt ist, kann, wie dargestellt, die Nadel 71 den durchstechbaren Dichtungsbereich 75 durchstechen, eine Sollbruchstelle kann zerbrechen, eine zerreißbare Membran kann zerreißen und/oder eine in Sperrrichtung vorgespannte Lippendichtung kann unter Überwindung der Vorspannung geöffnet werden, um eine Fluidverbindung zwischen dem mindestens einen Aufnahmebereich 21 und dem mindestens einen Kanal 25 freizugeben.

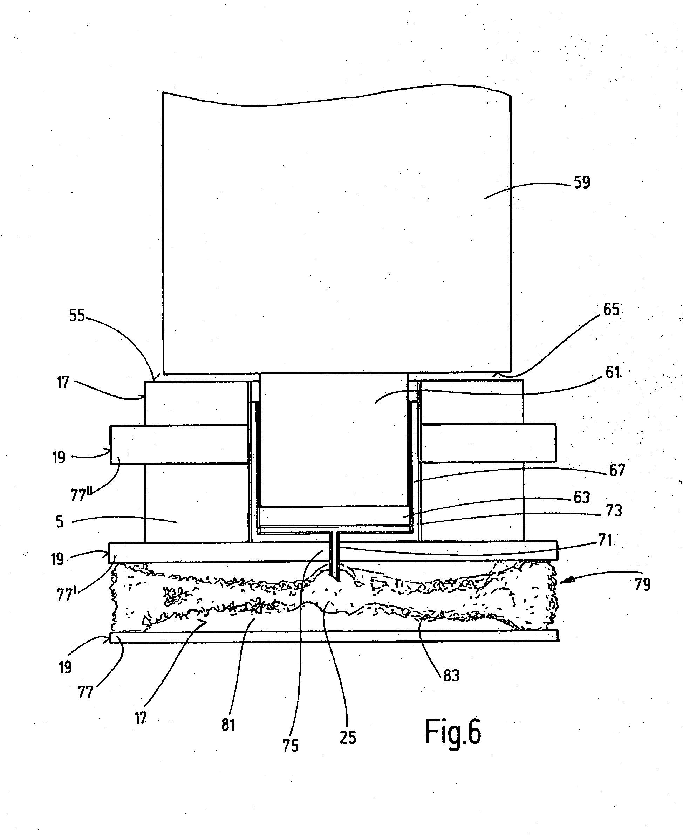

Figur 6 zeigt eine dritte Funktionsstellung des Kolbens 5 gemäß den Figuren 4 und 5, die ebenfalls dem zweiten Zustand zuzuordnen ist, wobei der Verbindungsbereich 61 der Kolbenstange 59 und die Dichtungseinrichtung 63 weiter in das Reservoir 67 hineinverlagert sind. Gleiche und funktionsgleiche Elemente sind mit gleichen Bezugszeichen versehen, sodass insofern auf die vorangegangene Beschreibung verwiesen wird. Die weitere Verlagerung der Kolbenstange 59 nach unten und insbesondere der Dichtungseinrichtung 63 beziehungsweise des Verbindungsbereichs 61 in das Reservoir 67 hinein bewirkt, dass das Gleitmittel aus dem Reservoir 67 über die Nadel 71, den hohlen Mittelbereich und den mindestens einen Kanal 25 in den Ringraum 83 ausgetrieben und damit an die mindestens eine Gleitfläche 19 abgegeben wird. Das in dem Bereich 79 angeordnete Gleitmittel ist hier gepunktet angedeutet. Das mindestens eine Abgabemittel, welches der Kolben 5 aufweist, umfasst bei dem in den Figuren 4, 5 und 6 dargestellten Ausführungsbeispiel also die als Abgabeelement ausgebildete Nadel 71, den nicht dargestellten hohlen Mittelbereich, den mindestens einen Kanal 25, sowie den Bereich 79 beziehungsweise den Ringraum 83. Diese Elemente stellen eine Fluidverbindung bereit, durch welche das Gleitmittel aus dem Reservoir 67 an die mindestens eine Gleitfläche 19 abgebbar ist.

Die durch die Kolbenstange 59 auf den Kolben 5 ausgeübte Kraft bewirkt zum Einen ein Austreiben des Gleitmittels aus dem Reservoir 67, zum Anderen aber auch eine Verlagerung des Kolbens 5. Vorzugsweise sind die Kräfteverhältnisse so austariert, dass bereits während des Austreibens des Gleitmittels aus dem Reservoir 67 auch eine Verlagerung des Kolbens 5 stattfindet. Bei einem anderen Ausführungsbeispiel ist es jedoch möglich, dass zunächst das Gleitmittel vollständig aus dem Reservoir 67 an den Ringraum 83 abgegeben wird, bevor dann, beispielsweise durch Anschlag der Anlagefläche 65 an der Oberfläche 55 des Kolbens 5 eine Verlagerung desselben erfolgt.

Es zeigt sich auch noch das Folgende: Auch bei dem in den Figuren 4, 5 und 6 dargestellten Ausführungsbeispiel eines Kolbens 5 ist es nicht zwingend erforderlich, dass die Druckkräfte über eine Kolbenstange 59 in den Kolben 5 eingeleitet werden. Bevorzugt ist es ebenso möglich, das Reservoir 67 nach oben, also zum oberen Teilraum 7 eines nicht dargestellten Hohlkörpers 1 hin mit einer elastischen und/oder für Gase durchlässigen Membran zu verschließen, sodass hier Druckkräfte aufgrund einer chemischen Reaktion einleitbar sind.

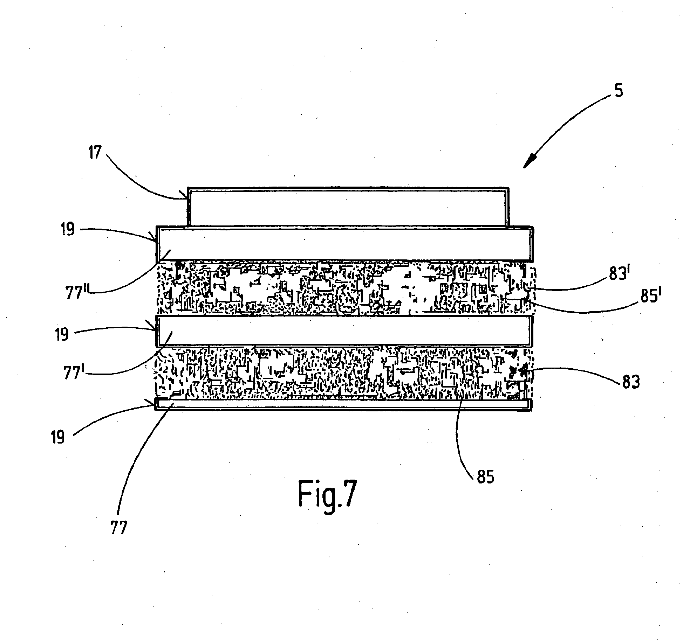

Figur 7 zeigt ein zweites Ausführungsbeispiel eines Kolbens 5. Gleiche und funktionsgleiche Elemente sind mit gleichen Bezugszeichen versehen, sodass insofern auf die vorangegangene Beschreibung verwiesen wird. Wie der Kolben 5 gemäß den Figuren 4 bis 6 weist auch das vorliegende Ausführungsbeispiel eine äußere Mantelfläche 17 auf, von der ausgehend sich in Umfangsrichtung gesehen umlaufende Vorsprünge 77, 77', 77" in radialer Richtung erstrecken, sodass hier Gleitflächen 19 gebildet werden, die einen größeren Außendurchmesser aufweisen als der Rest der im Wesentlichen zylindrischen, äußeren Mantelfläche 17. Es sind also auch hier Ringräume 83, 83' zwischen den Vorsprüngen 77 und 77', sowie den Vorsprüngen 77' und 77" ausgebildet. Der Kolben 5 weist auch hier mindestens einen Aufnahmebereich auf, welcher mindestens einen Schwamm, hier zwei ringförmig ausgebildete Schwämme 85, 85' umfasst, in deren poröser Struktur das mindestens eine Gleitmittel aufgenommen ist. Die ringförmig ausgebildeten Schwämme 85, 85' umgreifen eine äußere Umfangsfläche des Kolbens 5, hier die äußere Mantelfläche 17, zumindest bereichsweise.

Es ist offensichtlich, dass bei einem anderen bevorzugten Ausführungsbeispiel, welches nicht dargestellt ist, auch ein einziger Ringraum 83 mit einem Schwamm 85 vorgesehen sein kann. Bei wieder einem anderen Ausführungsbeispiel können auch mehr als zwei Ringräume 83, 83' mit mehr als zwei Schwämmen 85, 85' vorgesehen sein. Es ist auch möglich, den Ringraum 83 segmentartig zu unterteilen und in den entstehenden Teilräumen einzelne Schwammsegmente vorzusehen. Beliebige andere, zweckdienliche Anordnungen sind ebenfalls möglich. Vorzugsweise weisen die Schwämme 85, 85' einen Außendurchmesser auf, der größer als der Außendurchmesser des Kolbens 5 im Bereich der mindestens einen Gleitfläche 19 ist. Wird dann der Kolben 5 in den Innenraum 3 eines Hohlkörpers 1 eingebracht, werden die Schwämme 85, 85' komprimiert, sodass an ihrer an der inneren Mantelfläche 15 des Hohlkörpers 1 anliegenden Umfangsfläche Gleitmittel abgebbar ist. Diese Umfangsfläche dient hier also als Abgabemittel in Verbindung mit den die äußeren und inneren Poren verbindenden Kanälen, welche innerhalb des Schwammes ausgebildet sind. Wird der Kolben 5 innerhalb des Hohlkörpers 1 verlagert, hinterlassen die Schwämme 85, 85' einen Schmierfilm auf der inneren Mantelfläche 15, auf dem die Gleitflächen 19 gleiten können.

Es ist offensichtlich, dass der Kolben 5 gemäß Figur 7 sowohl durch mittels chemischer Reaktion erzeugte Druckkräfte als auch mit Hilfe einer Kolbenstange verlagert werden kann.

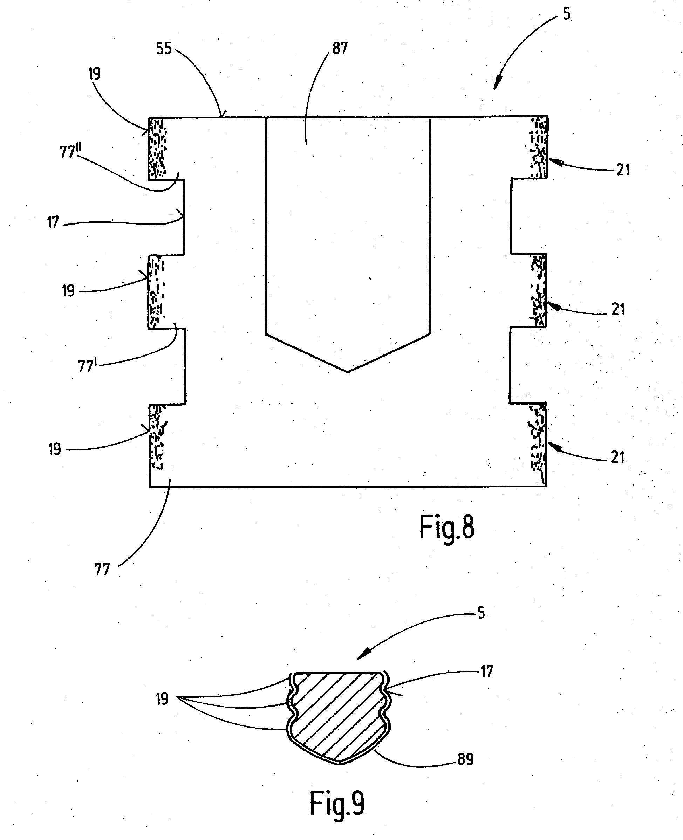

Figur 8 zeigt ein drittes Ausführungsbeispiel eines Kolbens 5. Gleiche und funktionsgleiche Elemente sind mit gleichen Bezugszeichen versehen, sodass insofern auf die vorangegangene Beschreibung verwiesen wird. Auch bei diesem Ausführungsbeispiel weist die äußere Mantelfläche 17 des Kolbens 5 Vorsprünge 77, 77', 77" auf, die Gleitflächen 19 umfassen. Vorzugsweise können auch mehr als drei Vorsprünge 77, 77', 77" vorgesehen sein. Bei einem anderen, nicht dargestellten Ausführungsbeispiel können aber auch nur zwei Vorsprünge 77, 77' vorgesehen sein. Nicht zuletzt ist es bei wieder einem anderen Ausführungsbeispiel möglich, die äußere Mantelfläche 17 - in axialer Richtung gesehen - mit konstantem Durchmesser durchgängig auszubilden, sodass kein Vorsprung 77, 77', 77" vorgesehen ist, und eine einzige Gleitfläche 19 durch die gesamte Mantelfläche 17 gebildet wird.

Das hier dargestellte Ausführungsbeispiel eines Kolbens 5 weist an den Gleitflächen 19 angeordnete Aufnahmebereiche 21 auf, welche mit Mikrobällchen durchsetzte Bereiche umfassen. Diese sind hier gepunktet dargestellt. Mikrobällchen sind vorzugsweise im Wesentlichen kugelförmige, kleine Kapseln, bei denen eine äußere Hülle einen Hohlraum umschließt, in dem ein Medium, hier Gleitmittel, aufnehmbar ist. Der Begriff "Mikrobällchen" spricht nicht notwendig an, dass diese Kapseln einen Durchmesser auf der Mikrometerskala aufweisen. Vorzugsweise können die Mikrobällchen zwar einen Durchmesser auf der Mikrometerskala aufweisen, es werden aber auch Ausführungsbeispiele bevorzugt, bei denen die Mikrobällchen einen hiervon abweichenden, insbesondere größeren Durchmesser aufweisen.

Besonders bevorzugt umfassen die Kapselhüllen das gleiche Material, welches auch der Kolben 5 im Bereich der Gleitfläche 19 umfasst. Ganz besonders bevorzugt besteht die Hülle der Kapseln beziehungsweise Mikrobällchen aus einem Elastomer. Die mit Gleitmittel gefüllten Mikrobällchen sind im Bereich der Gleitfläche 19 vorzugsweise in das Stopfenmaterial einvulkanisiert, also innig mit diesem verbunden. Durch die bei einer Verlagerung des Kolbens 5 auf die vorzugsweise dünne Hülle der Mikrobällchen wirkenden Reibungskräfte platzen diese auf und geben das von ihnen umfasste Gleitmittel an die Gleitfläche 19 ab, sodass hier im Kontaktbereich mit der inneren Mantelfläche 15 des Hohlkörpers 1 ein Schmierfilm gebildet wird. Die in dem Aufnahmebereich 21 angeordneten Mikrobällchen stellen also selbst das Abgabemittel dar, mit Hilfe dessen durch deren reibungsbedingtes Aufplatzen das Gleitmittel von dem mindestens einen Aufnahmebereich 21 an die mindestens eine Gleitfläche 19 abgebbar ist.

Es ist offensichtlich, dass auch der in Figur 8 dargestellte Kolben 5 sowohl durch aufgrund einer chemischen Reaktion freigesetzte Druckkräfte als auch mittels einer Kolbenstange 59 verlagerbar ist. Beispielhaft ist hier eine Ausnehmung 87 angedeutet, in die eine Kolbenstange 59 eingreifen kann. Es ist jedoch auch möglich, Reaktionsgase in die Ausnehmung 87 einzuleiten, sodass Druckkräfte nicht nur auf die Oberfläche 55 des Kolbens 5, sondern auch in der Ausnehmung 87 aufgrund der Reaktionsgase wirken, was schließlich zu einer Verlagerung des Kolbens 5 führt.

Figur 9 zeigt einen nicht zur Erfindung gehörenden Kolben 5, der keinen Aufnahmebereich für ein Gleitmittel und keine Abgabemittel aufweist. Er umfasst eine im Wesentlichen zylindrische äußere Mantelfläche 17, die hier - im Querschnitt gesehen - wellenförmig ausgebildet ist. Drei Teilflächen der Mantelfläche 17 bilden so Gleitflächen 19 zur Anlage an einem Hohlkörper 1, wie dies bereits in Zusammenhang mit Figur 1 erläutert wurde. Der hier dargestellte Kolben 5 umfasst zumindest im Bereich der mindestens einen Gleitfläche 19 ein glattes, unpolares Material. Vorzugsweise umfasst dieses glatte, unpolare Material PTFE, besteht besonders bevorzugt aus PTFE, welches besser unter dem Handelsnamen Teflon bekannt ist. Dieses Material weist besonders niedrige Haft- beziehungsweise Gleitreibungskoeffizienten auf, wodurch die Reibungskräfte zwischen der mindestens einen Gleitfläche 19 und der hier nicht dargestellten inneren Mantelfläche 15 eines Hohlkörpers 1 deutlich herabgesetzt werden können. Auf diese Weise ist es möglich, den Kolben 5 in dem Hohlkörper 1 unter vollständigem Verzicht auf ein Gleitmittel zu verlagern. Eine solche Verlagerung kann auch in diesem Fall selbstverständlich sowohl durch aufgrund einer chemischen Reaktion gebildete Druckkräfte als auch mit Hilfe einer Kolbenstange 59 erfolgen.

Bevorzugt ist der Kolben 5 zumindest im Bereich der mindestens einen Gleitfläche 19 mit einer Folie 89 beschichtet, die PTFE umfasst, vorzugsweise aus PTFE besteht.

Ein anderer, nicht dargestellter Kolben 5 besteht aus PTFE. Auch in diesem Fall ist die Reibung zwischen der mindestens einen Gleitfläche 19 und der inneren Mantelfläche 15 eines Hohlkörpers 1 deutlich reduziert.

Insgesamt zeigt sich, dass allen beschriebenen Ausführungsbeispielen eines Kolbens 5 und eines medizinischen Hohlkörpers 1 gemeinsam ist, dass in vorteilhafter Weise auf eine Beschichtung der inneren Mantelfläche 15 des Hohlkörpers 1 zumindest vor einer Ingebrauchnahme desselben verzichtet werden kann. So kann ausgeschlossen werden, dass eine in dem Innenraum 3 des Hohlkörpers 1 angeordnete pharmazeutische Substanz S vor allem während der Lagerung, bevorzugt aber auch während der Verwendung des Hohlkörpers 1 mit Gleitmittel, insbesondere Silikon, Silikonöl und/oder Silikonölemulsion in Kontakt kommt, wodurch eine Instabilität der pharmazeutischen Substanz S, insbesondere eine Aggregatbildung und/oder eine Niederschlagsfällung vermieden werden kann. Im Übrigen kann durch die Sperreinrichtung verhindert werden, dass ein in mindestens einem Aufnahmebereich vorhandenen Gleitmittel eine pharmazeutische Substanz im medizinischen Hohlkörper beeinträchtigt. Hierdurch können insbesondere auch unerwünschte Immunreaktionen bei einem Patienten, dem die pharmazeutische Substanz S injiziert wird, vermieden werden. Weiterhin ist eine Belastung des Organismus des Patienten durch zusammen mit der pharmazeutischen Substanz S injiziertes Gleitmittel ausgeschlossen.

## Patentansprüche

1. Kolben für einen medizinischen Hohlkörper (1), insbesondere eine Spritze oder Karpule, der mindestens eine Gleitfläche (19) zur Anlage an dem Hohlkörper (1) umfasst, außerdem mindestens einen Aufnahmebereich (21), in welchem ein Gleitmittel aufnehmbar ist, und mindestens ein Abgabemittel zur Abgabe des mindestens einen Gleitmittels von dem mindestens einen Aufnahmebereich (21) an die mindestens eine Gleitfläche (19), wobei der Aufnahmebereich (21) im Kolben (5) angeordnet ist, **dadurch gekennzeichnet, dass** der Kolben (5) mit einer Sperreinrichtung zusammenwirkt, wobei in einem ersten Zustand des Kolbens (5) kein Gleitmittel aus dem mindestens einen Aufnahmebereich (21) abgebbar ist, und dass in einem zweiten Zustand des Kolbens (5) Gleitmittel durch das mindestens eine Abgabemittel an die wenigstens eine Gleitfläche (19) abgebbar ist.

2. Kolben nach Anspruch 1, **dadurch gekennzeichnet, dass** der Kolben (5) in dem ersten Zustand drucklos und in dem zweiten Zustand mit einem Druck beaufschlagt ist.

3. Kolben nach Anspruch 1, **dadurch gekennzeichnet, dass** der mindestens eine Aufnahmebereich (21) mindestens ein Reservoir (67) einen Mikroballon und/oder einen mit Mikrobällchen durchsetzten Bereich umfasst.

4. Kolben nach Anspruch 1, **dadurch gekennzeichnet, dass** das mindestens eine Abgabemittel mindestens einen Kanal (25) umfasst, der von dem mindestens einen Aufnahmebereich (21) zu der mindestens einen Gleitfläche (19) führt, wobei in dem Kanal (25) Gleitmittel strömen kann.

5. Kolben nach Anspruch 1, **dadurch gekennzeichnet, dass** die Sperreinrichtung einen mit einer Nadel (71) durchstechbaren Bereich und eine Nadel (71), eine Sollbruchstelle, eine zerreißbare Membran und/oder eine in Sperrrichtung vorgespannte Lippendichtung umfasst.

6. Kolben nach Anspruch 1, **dadurch gekennzeichnet, dass** der Kolben (5) ein Elastomer umfasst, vorzugsweise aus einem Elastomer besteht.

7. Kolben nach Anspruch 1, **dadurch gekennzeichnet, dass** der Kolben (5) PTFE umfasst, vorzugsweise aus PTFE besteht.

8. Kolben nach Anspruch 1, **dadurch gekennzeichnet, dass** das Gleitmittel Silikon, ein Silikonöl und/oder eine Silikonölemulsion umfasst.

9. Kolben nach Anspruch 1, **dadurch gekennzeichnet, dass** das Sperrmittel mindestens eine Membran (27) umfasst, die den mindestens einen Aufnahmebereich (21) zumindest bereichsweise verschließt.

10. Kolben nach Anspruch 9, **dadurch gekennzeichnet, dass** die Membran (27) elastisch und/oder für Gase durchlässig ausgebildet ist.

11. Medizinischer Hohlkörper, insbesondere Spritze oder Karpule, mit mindestens einem in einem Innenraum (3) des Hohlkörpers (1) entlang dessen Längsachse (L) verlagerbaren Kolben (5), **dadurch gekennzeichnet, dass** der Kolben (5) ein Kolben (5) nach einem der Ansprüche 1 bis 10 ist.

12. Medizinischer Hohlkörper nach Anspruch 11, **dadurch gekennzeichnet, dass** der Hohlkörper (1) zusätzlich eine Vorrichtung (29) umfasst, mittels derer Druckkräfte auf den mindestens einen Kolben (5) durch eine chemische Reaktion ausübbar sind.

13. Medizinischer Hohlkörper nach Anspruch 11, **dadurch gekennzeichnet, dass** der Hohlkörper (1) eine Kolbenstange (59) umfasst, mittels derer Druckkräfte auf den mindestens einen Kolben (5) ausübbar sind.

## Claims

1. Piston for a medical hollow body (1), in particular for a syringe or a carpule, the piston comprising at least one sliding surface (19) for contact with the hollow body (1), furthermore at least one receiving area (21) into which a lubricant can be received, and at least one dispensing means for dispensing the at least one lubricant from the at least one receiving area (21) to the at least one sliding surface (19), the receiving area (21) being arranged within the piston (5), **characterized in that** the piston (5) cooperates with a blocking device, wherein in a first state of the piston (5), no lubricant can be dispensed from the at least one receiving area (21), and that in a second state of the piston (5), lubricant can be dispensed by the at least one dispensing means to the at least one sliding surface (19).

2. Piston according to claim 1, **characterized in that** the piston (5) is unpressurized in the first state and is subjected to pressure in the second state.

3. Piston according to claim 1, **characterized in that** the at least one receiving area (21) comprises at least one reservoir (67), one microballoon and/or one area penetrated with microballs.

4. Piston according to claim 1, **characterized in that** the at least one dispensing means comprises at least one channel (25) which runs from the at least one receiving area (21) to the at least one sliding surface (19), wherein lubricant can flow in the channel (25).

5. Piston according to claim 1, **characterized in that** the blocking device comprises an area that is piercable with a needle (71), and a needle (71), a predetermined breaking point, a tearable diaphragm, and/or a lip seal pretensioned in the blocking direction.

6. Piston according to claim 1, **characterized in that** the piston (5) comprises an elastomer and preferably consists of an elastomer.

7. Piston according to claim 1, **characterized in that** the piston (5) comprises PTFE and preferably consists of PTFE.

8. Piston according to claim 1, **characterized in that** the lubricant comprises silicone, a silicone oil, and/or a silicone oil emulsion.

9. Piston according to claim 1, **characterized in that** the blocking device comprises at least one diaphragm (27) which closes the at least one receiving area (21) at least in certain areas.

10. Piston according to claim 9, **characterized in that** the diaphragm (27) is formed to be elastic and/or permeable for gases.

11. Medical hollow body, in particular a syringe or a carpule, comprising at least one piston (5) which can be displaced within an interior (3) of the hollow body (1) along the longitudinal axis (L) of said hollow body, **characterized in that** the piston (5) is a piston (5) according to any one of the claims 1 to 10.

12. Medical hollow body according to claim 11, **characterized in that** the hollow body (1) comprises in addition a device (29) by means of which pressure forces due to a chemical reaction can be exerted on the at least one piston (5).

13. Medical hollow body according to claim 11, **characterized in that** the hollow body (1) comprises a piston rod (59) by means of which pressure forces can be exerted on the at least one piston (5).

## Revendications

1. Piston pour un corps creux (1) pour le domaine médical, notamment une seringue ou une carpule, comprenant au moins une surface de glissement (19) pour rester contre le corps creux (1), en outre au moins une zone de réception (21) qui peut recevoir un lubrifiant, et au moins un moyen de délivrance pour délivrer le au moins un lubrifiant de la au moins une zone de réception (21) sur la au moins une surface de glissement (19), la zone de réception (21) étant disposée dans le piston (5), **caractérisé en ce que** le piston (5) coopère avec un dispositif de blocage, dans lequel, dans un premier état du piston (5), pas de lubrifiant peut être délivré de la au moins une zone de réception (21), et **en ce que**, dans un second état du piston (5), du lubrifiant peut être délivré du au moins un moyen de délivrance sur la au moins une surface de glissement (19).

2. Piston selon la revendication 1, **caractérisé en ce que** le piston (5) n'est pas sous pression dans le premier état, et est mis sous une pression dans le second état.

3. Piston selon la revendication 1, **caractérisé en ce que** la au moins une zone de réception (21) comprend au moins un réservoir (67), un microballon et/ou une zone chargées par des microsphères.

4. Piston selon la revendication 1, **caractérisé en ce que** le au moins un moyen de délivrance comprend au moins un conduit (25) qui s'étend de la au moins une zone de réception (21) à la au moins une surface de glissement (19), dans lequel le lubrifiant peut s'écouler dans le conduit (25).

5. Piston selon la revendication 1, **caractérisé en ce que** le dispositif de blocage comprend une zone qui peut être percée par une aiguille (71) aussi bien qu'une aiguille (71), une zone de rupture imposée, une membrane déchirable et/ou un joint d'étanchéité à lèvre précontraint dans la direction de blocage.

6. Piston selon la revendication 1, **caractérisé en ce que** le piston (5) comprend un élastomère, et de préférence est constitué d'un élastomère.

7. Piston selon la revendication 1, **caractérisé en ce que** le piston (5) comprend du PTFE, et de préférence est constitué du PTFE.

8. Piston selon la revendication 1, **caractérisé en ce que** le lubrifiant comprend du silicone, d'une huile de silicone et/ou d'une émulsion à base d'huile de silicone.

9. Piston selon la revendication 1, **caractérisé en ce que** le moyen de blocage comprend au moins une membrane (27) qui ferme, d'au moins partiellement, la au moins une zone de réception (21).

10. Piston selon la revendication 9, **caractérisé en ce que** la membrane (27) est élastique et/ou configurée d'être perméable aux gaz.

11. Corps creux pour le domaine médical, notamment une seringue ou une carpule, avec au moins un piston (5) déplaçable dans un intérieur (3) du corps creux (1) le long de son axe longitudinal (L), **caractérisé en ce que** le piston (5) est un piston (5) selon l'une quelconque des revendications 1 à 10.

12. Corps creux pour le domaine médical selon la revendication 11, **caractérisé en ce que** le corps creux (1) comprend en outre un dispositif (29) par lequel des forces de pression peuvent être appliquées sur le au moins un piston (5) par une réaction chimique.

13. Corps creux pour la domaine médicale selon la revendication 11, **caractérisé en ce que** le corps creux (1) comprend une tige de piston (59) par laquelle des forces de pression peuvent être appliquées sur le au moins un piston (5).
